# FASCICULE DE BREVET EUROPEEN

(11) **EP 1 243 586 B1**
(45) Date de publication et mention de la délivrance du brevet: **14.09.2005**
(21) Numéro de dépôt: 02290690.3
(22) Date de dépôt: 19.03.2002
(51) Int. Cl.: C07D 311/62, A61K 7/48

(54) **Fraction phénolique riche en phloridzine et son utilisation en tant qu'agent cosmétique, alimentaire ou nutraceutique.**
Phlorizinreiche Phenolfraktion und ihre Verwendung in der Kosmetik oder als Nahrungsmittel
Phloridzin-rich phenolic fraction and its use as cosmetic, food or nutritional agent

(30) Priorité: 23.03.2001 FR 0103968
(43) Date de publication de la demande: 25.09.2002
(73) Titulaire: Diana Ingredients, 56250 Elven (FR)
(72) Inventeur: Gaudout, David, 35300 Fougères (FR); Megard, Denis, 35460 Saint-Brice-en-Cogles (FR); Inisan, Claude, 35000 Rennes (FR); Esteve, Christian, 53230 Cosse-le-Vivien (FR); Lejard, Frédéric, 56610 Arradon (FR)
(74) Mandataire: Casalonga, Axel

(56) Documents cités:
- EP-A- 0 657 169
- G. PAGANGA ET AL.: "THE POLYPHENOLIC CONTENT OF FRUIT A. VEGETABLES AND THEIR ANTIOXIDANT ACTIVITIES." FREE RAD. RES., vol. 30, no. 2, 1999, pages 153-62, XP001041471 MALAYSIA

## Description

La présente invention concerne une fraction phénolique de fruits ainsi que le procédé d'obtention de cette fraction. Cet extrait riche en un composé anti-oxydant, la phloridzine, est utilisable en tant que préparation cosmétique, alimentaire ou nutraceutique.

Il est connu que les composés polyphénoliques sont assez répandus et en quantité importante dans le règne végétal. Dans la famille des *Rosaceae* en particulier, l'analyse des polyphénols de la pomme a conduit à l'identification d'au moins 37 composés phénoliques dont les plus abondants sont l'acide chlorogénique, les procyanidines B1 et B2, l'épicatéchine, la phlorétine, la phloridzine et l'acide p-coumarique. Certains de ces composés possèdent des propriétés physiologiques telles que des propriétés anti-oxydantes, anti-mutagènes, anti-allergiques, anti-cancéreuses, anti-diabétiques et d'autres.

De nombreux autres produits riches en polyphénols existent sur le marché, les plus courants étant extraits du thé vert, des pépins de raisin et de l'écorce de pin (US A 4 698 360, EP A 348 781, EP A 283 349, FR A 1 427 100, FR A 2 092 743, FR A 2 643 073 et FR A 2 372 823). Il a déjà été décrit dans le brevet EP A 0 657 169 l'extraction d'une fraction polyphénolique à partir de fruits non matures (pesant de 3 à 10 grammes) de la famille des rosaceae. La fraction polyphénolique ainsi définie se caractérise par une haute teneur en dérivés de la famille des acides hydroxycinnamiques (acide chlorogénique, acide cafféique, acide p-coumarique), et en molécules de la famille des flavanols (catéchine, épicatéchine et procyanidine). L'analyse par chromatographie liquide à haute performance d'un extrait issu de jus de fruits immatures montre que la phloridzine représente moins de 7 % en poids de l'ensemble des composés phénoliques et les dihydrochalcones (phloridzine et phlorétine) moins de 9 %.

Parmi les composés phénoliques, la phlorétine et son dérivé glycosylé, la phloridzine sont typiques de la pomme et des autres fruits de la famille des Rosaceae. On trouve en particulier la phloridzine en quantité importante dans les pépins, mais elle est également présente dans les jus et la peau de pomme. La phloridzine possède une activité anti-oxydante permettant une protection cardiovasculaire similaire à celles des oestrogènes. D'autre part, la phloridzine est capable d'agir sur la mélanogénèse par activation d'une cascade d'enzymes dont la tyrosinase, permettant ainsi une protection accrue vis-à-vis des rayonnements ultraviolets. La phloridzine présente également une action anti-diabétique par inhibition compétitive du transport sanguin sodium-dépendant des métabolites tels que le glucose, le galactose et autres. La phloridzine intervient également dans l'inhibition de la croissance des cellules tumorales par blocage de l'activité protéine kinase C.

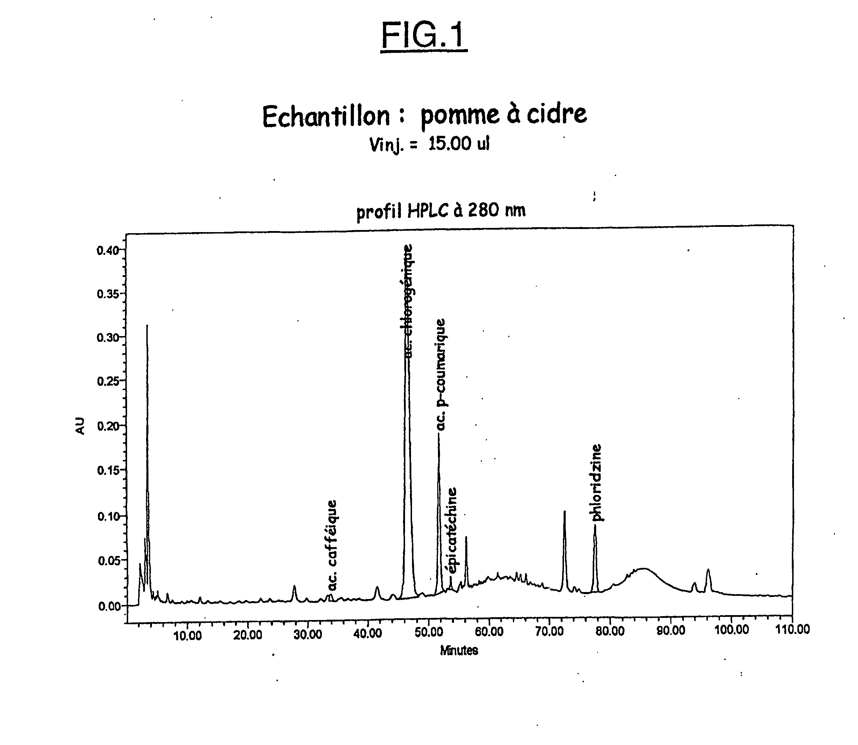

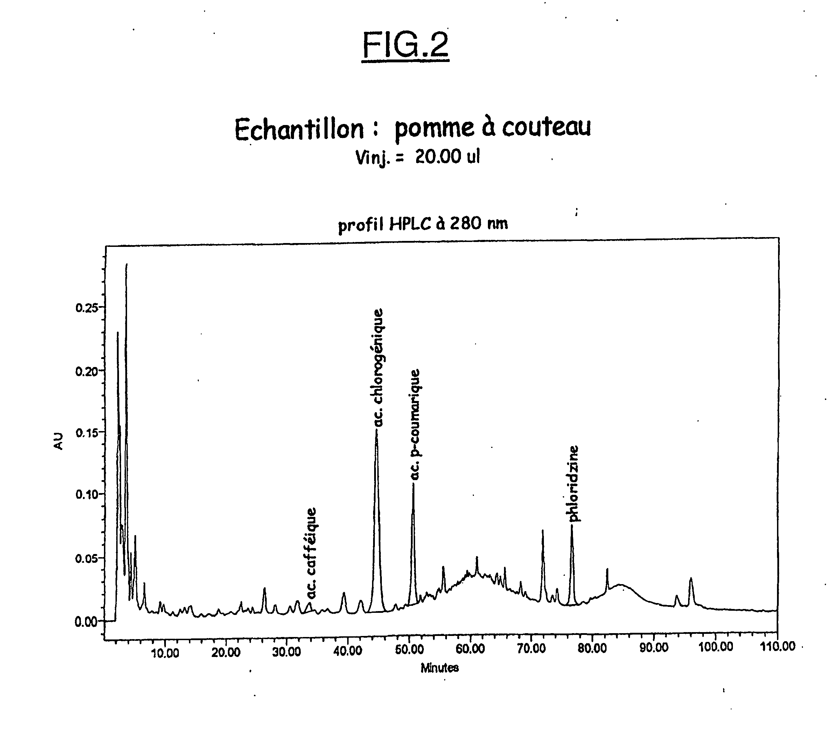

Cependant, dans la pomme (broyat ou jus), les dihydrochalcones (phlorétine et phloridzine) sont présentes en quantité mineure par rapport aux autres polyphénols. L'acide chlorogénique et les procyanidines sont les polyphénols majoritaires des pommes, que ce soit des pommes "à cidre" ou "à couteau", la phloridzine et la phlorétine ne représentant jamais plus de 5 % en poids des polyphénols totaux de pommes à cidre mûres (analyse de 15 variétés différentes) (figures 1 et 2).

Dans les extraits polyphénoliques connus, les proportions entre les différentes molécules phénoliques sont conservées par rapport aux proportions présentes dans les différentes matières premières, à l'exception des procyanidines polymériques qui sont perdues ou dégradées lors de l'extraction :

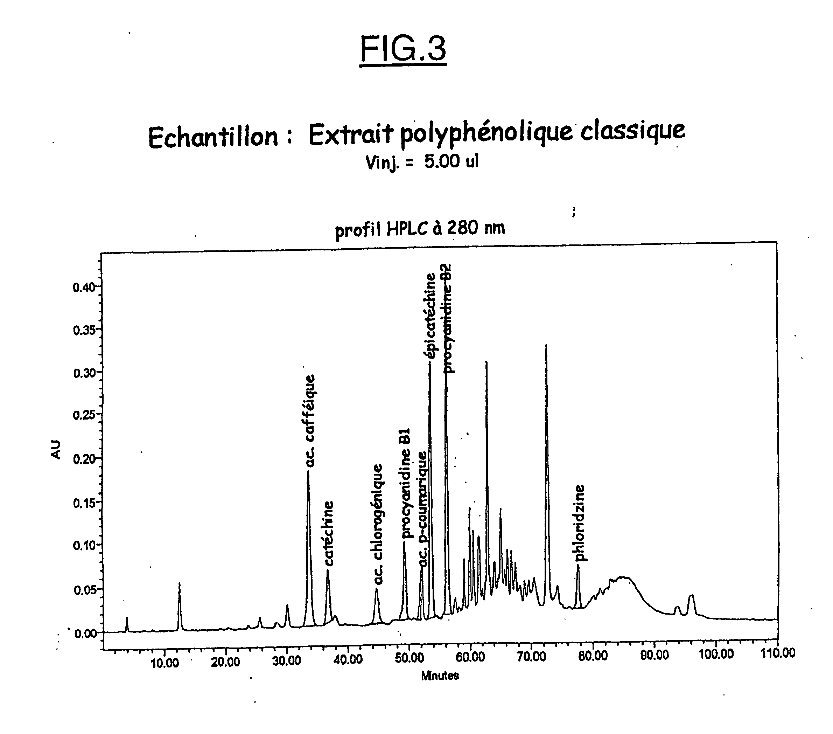

On obtient donc classiquement des extraits polyphénoliques riches en acides hydroxycinnamiques (acides cafféique, chlorogénique et coumarique) et en flavanols (catéchine, épicatéchine et procyanidines), et pauvres en dihydrochalcones (phloridzine et phlorétine) (voir figure 3).

| Composé phénolique | Pomme * en mg/L de jus ou kg de broyat | Extrait polyphénolique connu de pomme (en mg/g poudre) |
|---|---|---|
| Acide cafféique | ε | 21.7 |
| Catéchine | ε à 150 | 15.1 |
| Acide chlorogénique | 60 à 1200 | 161.0 |
| Procyanidines | 500 à 5000 | 69.6 (B ₁ et B ₂ ) |
| Acide p-coumarique | 1 à 150 | 9.3 |
| Epicatéchine | ε à 1400 | 41.4 |
| Phloridzine | 6 à 100 | 32.7 |
| Quercitrine | ε | 1.9 |
| Phlorétine | 5 à 100 | 9.5 |
| **Polyphénols totaux** (exprimés en équivalent phloridzine) | | **483.4** |

| | | |
|---|---|---|
| ε = quantité non quantifiable | | |
| * Valeurs compilées de mesures sur 15 variétés de pommes à cidre et 3 variétés de pommes à couteau sur 3 campagnes. Karadeniz F & Ekski A. Phenolic compounds in apple juice from different varieties Report - *Scientific Technical Com. Int. Fed. Fruit Juice Producers*, (1996), **24**, pp 265-275. Sanoner P., Guyot S., Marnet N. et Drilleau J.F. Polyphenolic profiles of French cider apples varieties. Dans 'Polyphenols, wines and health', symposium, Bordeaux, (14-16 avril 1999). Sanoner P., Guyot S., Marnet N., Molle D. et Drilleau J.F. Polyphenol profiles of French cider apples varieties. *J. Agric. Food Chem.* (1999), **47**, pp 4847-4853. | | |

Il n'y a que très peu, voire pas du tout, d'acide cafféique naturellement dans les pommes. L'acide cafféique est en fait probablement un produit de dégradation de l'acide chlorogénique (Fiedler, 1954, Arzneimittel-Forsch, 4, 41).

La demanderesse a mis au point de nouvelles fractions phénoliques, qui constituent l'objet de l'invention.

L'invention a également comme objet le procédé d'obtention de cette fraction.

Un autre objet est constitué par les applications de cette fraction comme complément alimentaire, nutraceutique ou comme moyen de protection contre les rayonnements ultraviolets, comme composition cosmétique.

D'autres objets apparaîtront à la lecture de la description et des exemples qui suivent.

La fraction polyphénolique conforme à l'invention, comprend au moins 20 % en poids de polyphénols et de préférence 50 % dont 10 % au moins en poids est composé de phloridzine et de préférence entre 10 et 70 %. Cet extrait peut comprendre également dans sa composition de l'acide chlorogénique, de l'épicatéchine, de la procyanidine B2, de la quercitrine, de l'acide p-coumarique ainsi que de la phlorétine.

Une composition particulièrement préférée de cette fraction polyphénolique est qu'elle contient en poids :
- plus de 20 % de polyphénols totaux, préférentiellement plus de 50 %
- au moins 30 % des polyphénols en phloridzine et de préférence de 30 à 40 % en poids
- au plus 11 % des polyphénols en acide chlorogénique et de préférence entre 2 et 11 % en poids
- au plus 4 % des polyphénols en épicatéchine
- au plus 2 % des polyphénols en Procyanidine B2
- au plus 1,5 % des polyphénols en quercitrine
- au plus 0,4 % des polyphénols en acide p-coumarique
- et moins de 0,2 % des polyphénols en acide cafféique

Un autre objet de l'invention est que l'acide cafféique est présent dans des proportions en poids inférieures à 20 % du poids de phloridzine présent. De préférence, l'acide cafféique représente moins de 1 % en poids des polyphénols totaux des extraits. La proportion de phloridzine est 9 fois supérieure en poids à celle de la catéchine. La quantité de phloridzine présente est au moins équivalente en poids à celle de l'acide chlorogénique.

Un autre objet de l'invention est caractérisé par le fait qu'elle contient de la phlorétine. Par une hydrolyse acide ménagée, on peut transformer la quasi-totalité de la phloridzine en phlorétine, moins soluble dans l'eau.

Un autre objet est caractérisé par le fait que les dihydrochalcones sont présentes dans des proportions supérieures ou égales à 40 % en poids par rapport aux acides hydroxycinnamiques.

Le procédé d'extraction permettant l'extraction sélective d'une fraction polyphénolique riche en dihydrochalcones à partir de pommes mûres est caractérisé par le fait qu'il comporte les étapes suivantes :
■ Les pommes broyées font l'objet d'une ou plusieurs extractions solide / liquide, en présence ou non d'eau ajoutée.
■ L'extrait solide humide obtenu est ensuite soit séché, soit liquéfié enzymatiquement pour obtenir un extrait liquide.
■ L'extrait solide sec subit de nouvelles extractions pendant un temps compris entre 10 minutes et 2 heures par un solvant organique polaire, de préférence un alcool aliphatique en C1-C4, pur ou en mélange avec de l'eau pour obtenir un extrait organique.
■ Celui-ci est évaporé à sec à une température inférieure ou égale à 60 °C de préférence sous pression réduite.
■ Ce résidu est ensuite repris par l'eau avant d'être épuisée plusieurs fois, de préférence 4 fois, par un solvant non miscible à l'eau, de préférence l'acétate d'éthyle ou l'acétate de méthyle ou de propyle.
■ Les solutions organiques obtenues sont mélangées et évaporées à sec à une température inférieure à 60 °C, préférentiellement inférieure à 50 °C, pour obtenir la fraction polyphénolique qui fait l'objet de la présente invention.
■ Par une autre voie, l'extrait solide humide est mélangé à de l'eau en présence d'un mélange enzymatique pendant un temps compris entre 1 et 4 heures à une température comprise entre 30 et 50°C, préférentiellement entre 40 et 45°C, pour obtenir un extrait liquide.
■ Cet extrait liquide est clarifié par centrifugation ou par filtration puis par ultrafiltration.
■ L'extrait est chargé sur une colonne chromatographique remplie d'une résine adsorbante de type styrène-divinylbenzène. La résine est lavée avec de l'eau acidifiée pour éliminer les impuretés et les sucres résiduels. Les polyphénols sont ensuite élués par une solution hydro-alcoolique renfermant entre 40 et 70 % d'éthanol préférentiellement entre 50 et 60 % en poids. D'autres alcools aliphatiques en C1-C4 peuvent être utilisés tels que le méthanol ou le butanol.
■ Si nécessaire, une étape de dé-cirage est introduite en cours de procédé.
■ Le produit obtenu par extraction est repris une dernière fois à l'eau avant d'être séché de préférence par atomisation ou lyophilisation pour obtenir une poudre beige contenant au moins 20 % en poids de polyphénols, préférentiellement plus de 50 % de polyphénols, et dont 10 % en poids et préférentiellement entre 10 et 70 % des polyphénols sont des dihydrochalcones, préférentiellement de la phloridzine.

Les fractions sont obtenues par le procédé précédemment décrit de préférence à partir de pommes matures de la famille des rosaceae et en particulier de l'espèce Malus sylvestris Mill.

Cet extrait de pommes comestibles possédant les caractéristiques énoncées, obtenu selon le procédé précédemment décrit, est utilisable en tant que complément alimentaire ou nutraceutique.

La fraction phénolique riche en dihydrochalcones selon l'invention présente des propriétés comme agent de protection vis-à-vis des rayonnements ultraviolets.

La fraction phénolique riche en dihydrochalcones selon l'invention présente des propriétés comme agent anti-oxydant.

L'invention a également pour objet une composition cosmétique comprenant, entre autres, la fraction polyphénolique riche en dihydrochalcones précédemment décrite.

Les compositions selon l'invention peuvent être ingérées ou appliquées sur la peau. Selon le mode d'administration, la composition selon l'invention peut se présenter sous toutes les formes habituellement utilisées en cosmétique.

Les compositions selon l'invention peuvent notamment se présenter sous forme de gélules, de laits, de lotions, de crèmes, de gels ou de boissons.

Les compositions nutraceutiques, alimentaires ou cosmétiques de la présente invention sont formulées classiquement selon les applications auxquelles elles sont destinées.

Les exemples suivants illustrent l'invention sans la limiter aucunement.

### Exemple 1 : Protocole n°1 d'extraction de la fraction polyphénolique de pomme

On a soumis des pommes de l'espèce Malus sylvestris Mill. au traitement d'extraction suivant :
■ les pommes sont broyées et le broyat est agité en présence de 20 % d'eau environ.
■ le broyat fait l'objet d'extractions solide / liquide successives pour éliminer les fractions liquides riches en sucre.
■ l'extrait solide encore humide de la deuxième étape est séché puis traité pendant une durée de 2 h dans un mélange méthanol/eau en proportions 90/10 en poids.
■ l'extrait organique est évaporé à sec à une température d'environ 50 °C, sous pression réduite.
■ le résidu d'évaporation est repris par l'eau, puis la solution aqueuse obtenue est épuisée 4 fois, par l'acétate d'éthyle, solvant non miscible à l'eau.
■ les solutions organiques obtenues sont mélangées et évaporées à une température d'environ 45 °C pour obtenir un extrait sec.
■ l'extrait sec est repris une dernière fois à l'eau pour un séchage final, de préférence par atomisation ou lyophilisation, et obtenir une poudre fine de couleur beige partiellement soluble dans l'eau.

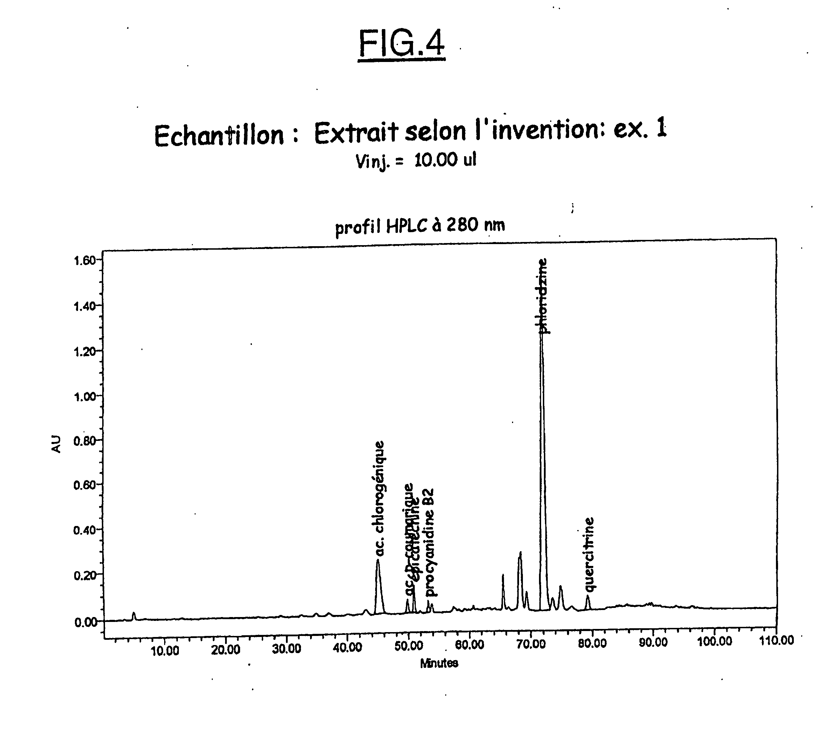

La composition en polyphénols de cet extrait, en mesurant par chromatographie liquide à haute performance la concentration de chaque molécule par rapport à son standard pur est la suivante (figure 4).

Dans chaque extrait ont ainsi été mesurées les concentrations de :

| Composés phénoliques | Extrait polyphénolique selon l'invention (en mg/g) |
|---|---|
| Polyphénols totaux (exprimés en équivalent phloridzine) | 492.1 |
| Phloridzine | 196.2 |
| Acide chlorogénique | 55.0 |
| Epicatéchine | 20.8 |
| Procyanidine B2 | 11.3 |
| Quercitrine | 7.9 |
| Acide p-coumarique | 2.3 |
| Acide cafféique | <1 |

On constate que la fraction phénolique obtenue par ce procédé d'extraction est particulièrement riche en phloridzine. Elle présente la caractéristique d'être pauvre en acides de la famille des acides hydroxycinnamiques, et plus particulièrement en acide chlorogénique. Elle est également pauvre en acides de la famille des flavanols (catéchines, épicatéchines, procyanidines B1, B2...), ce qui conduit à un profil phénolique original totalement différent de ceux décrits pour la pomme, et totalement différent d'un profil phénolique obtenu par un procédé classique à partir de pommes mûres.

La poudre obtenue titre 55 % de polyphénols totaux en poids par la méthode colorimétrique de Folin-Ciocalteu, en utilisant l'acide chlorogénique comme standard. Cette méthode utilise le réactif de Folin-Ciocalteu (référence 9001, Merck). Brièvement, ce réactif est constitué d'un mélange d'acide phosphotungstique et d'acide phosphomolybdique, qui est réduit lors de l'oxydation des phénols en un mélange d'oxydes bleus de tungstène et de molybdène. La couleur développée est proportionnelle au taux de composés phénoliques présents.

Par méthode chromatographique et intégration du profil obtenu par chromatographie liquide à haute performance, elle titre entre 49 et 67 % de polyphénols totaux en poids selon que l'on exprime la valeur en équivalent phloridzine et équivalent acide chlorogénique respectivement.

### Exemple 2 : Protocole n°2 d'extraction de la fraction polyphénolique de pomme

On a soumis des pommes de l'espèce Malus sylvestris Mill. au traitement d'extraction suivant :
■ les pommes sont broyées et le broyat est agité en présence d'environ 20 % d'eau.
■ le broyat fait l'objet d'extractions solide / liquide successives pour éliminer les fractions liquides enrichies en sucre.
■ l'extrait solide humide est mélangé à de l'eau en présence d'un mélange enzymatique riche en activités pectolytiques, hémicellulasiques et cellulasiques pendant environ 2 heures à une température d'environ 45 °C pour obtenir un extrait liquide.
■ l'extrait liquide est clarifié par centrifugation ou par filtration puis par ultrafiltration.
■ l'extrait liquide limpide est ensuite chargé sur une colonne chromatographique remplie d'une résine adsorbante de type styrène-divinylbenzène.
■ la résine est lavée avec de l'eau acidifiée pour éliminer les impuretés et les sucres résiduels.
■ les polyphénols sont ensuite élués par une solution hydro-alcoolique renfermant 60 % en poids d'éthanol.
■ la solution polyphénolique obtenue est concentrée puis séchée par atomisation ou lyophilisation pour obtenir une poudre fine beige.

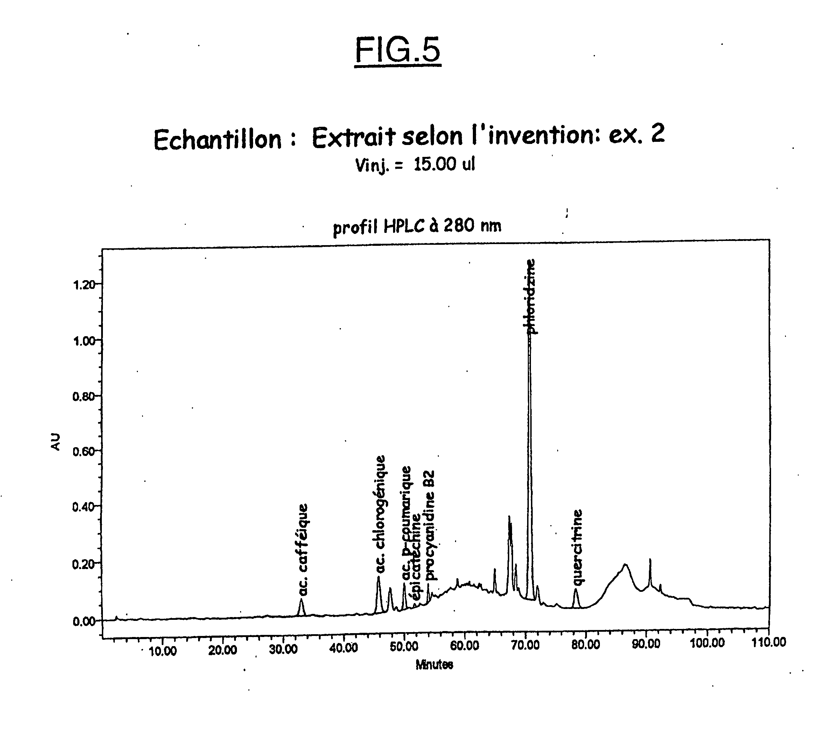

La composition en polyphénols de cet extrait, en mesurant par chromatographie liquide à haute performance la concentration de chaque molécule par rapport à son standard pur est la suivante (figure 5) :

| Composés phénoliques | Extrait polyphénolique selon l'invention ( en mg/g) |
|---|---|
| Polyphénols totaux (exprimés en équivalent phloridzine) | 293.2 |
| Phloridzine | 36.7 |
| Acide chlorogénique | 7.5 |
| Epicatéchine | 1.0 |
| Procyanidine B2 | ε |
| Quercitrine | 3.8 |
| Acide p-coumarique | 1.2 |
| Acide cafféique | 1.7 |

La poudre obtenue titre 50 % de polyphénols totaux en poids par la méthode colorimétrique de Folin-Ciocalteu, en utilisant l'acide chlorogénique comme standard.

On constate que la fraction phénolique obtenue par ce procédé d'extraction est particulièrement riche en phloridzine. Comme l'extrait de l'exemple 1, cette fraction présente la caractéristique d'être pauvre en acides de la famille des acides hydroxycinnamiques et plus particulièrement en acide chlorogénique. Elle présente également la caractéristique d'être pauvre en flavanols (catéchines, épicatéchines, procyanidines B1, B2..), ce qui conduit à un profil phénolique original totalement différent de ceux décrits pour la pomme, et totalement différent d'un profil phénolique obtenu par un procédé classique à partir de pommes mûres.

Contrairement à l'exemple 1, une petite quantité d'acide cafféique est présente, ne représentant que 4 % en poids de la quantité de phloridzine présente.

Par intégration du profil obtenu par chromatographie liquide à haute performance, elle titre entre 29 et 40 % en poids de polyphénols totaux selon que l'on exprime la valeur en équivalent phloridzine et en équivalent acide chlorogénique respectivement.

### Exemple 3 : Protocole n°3 d'extraction de la fraction polyphénolique de pomme

L'extrait polyphénolique riche en phloridzine selon l'invention peut être modifié avant l'étape de séchage final. Le procédé consiste en une hydrolyse acide ménagée en présence d'acide minéral (dilué à un pH d'environ 3) de la phloridzine (phlorétine 2'-β-glucoside) en phlorétine. La poudre obtenue après séchage est peu soluble dans l'eau (très faible solubilité de la phlorétine) mais présente une activité anti-oxydante intéressante.

### Exemple 4 : Activité anti-oxydante (ou anti-radicalaire) de la fraction polyphénolique riche en phloridzine selon l'invention :

La mesure de l'activité anti-radicalaire des extraits polyphénoliques est réalisée par la méthode du DPPH (1,1-diphényl-2-picrylhydrazyl) décrite par Brand-Williams et al. (1995) et Lu et Foo (2000) légèrement modifiée. Brand-Williams W., Cuvelier M.E. & Berset C. (1995) Use of a free radical method to evaluate antioxidant activity. *Lebensmittel-Wissenschaft und Technologie,* **28,** pp 25-30. Lu Y. & Foo L.Y. ((2000) Antioxidant and radical scavenging activities of polyphenols from apple pomace. *Food Chemistry,* **68,** pp 81-85.

Le principe est de mesurer la quantité résiduelle de DPPH°, radical coloré stable dans les conditions opératoires et dont la concentration diminue lors d'une incubation en présence d'extraits anti-oxydants dans des conditions standardisées. En effet, les extraits présentant une activité anti-oxydante réduisent le radical DPPH° en DPPH₂. L'activité anti-oxydante est exprimée par rapport à l'activité d'une molécule de référence, le trolox (analogue hydrosoluble de la vitamine E).

6 mg de DPPH° sont soigneusement dissous dans 100 mL d'une solution aqueuse de méthanol à 75 % en vol. En parallèle, une quantité précise d'échantillon à analyser et 18 mg de Trolox sont dissous dans 25 mL de solution méthanolique à 75 %. On incube 2 mL de solution de DPPH° avec 25 µL de solution à mesurer à une température de 37 ± 0.5°C dans des flacons étanches pendant 6 heures. La diminution de l'intensité colorante de la solution est mesurée à une longueur d'onde de 515 nm à t=0 min, t=5 min. et t=6 heures. L'activité est exprimée en mmole équivalent trolox / kg de produit après 6 h d'incubation.

La méthode du DPPH a été employée pour déterminer l'activité anti-oxydante de ces produits :
■ Plasma sanguin
■ Vin rouge
■ Extrait concentré de vin rouge à 5 % de polyphénols totaux
■ Poudre de vin rouge à 55 % de polyphénols totaux
■ Jus de pomme
■ Extrait de sureau à 40 % de polyphénols totaux (DO 280 nm)
■ Extrait de myrtille à 30 % de polyphénols totaux (DO 280 nm)
■ Extrait polyphénolique à 50 % de polyphénols totaux riche en phloridzine selon le procédé de l'invention
■ Extrait polyphénolique à 90 % de polyphénols totaux riche en phloridzine selon le procédé de l'invention
■ Extrait polyphénolique à 90 % de polyphénols totaux riche en phlorétine selon le procédé de l'invention

| Produit | Capacité anti-oxydante (mmole équivalent de trolox/kg de produit) |
|---|---|
| Plasma sanguin (référence biologique) | 20-25 |
| Vin rouge | 15-25 |
| Extrait concentré de vin rouge à 5% de polyphénols totaux | 680-700 |
| Jus de pomme | 5-20 |
| Extrait de sureau à 40 % de polyphénols totaux en poids | 2900-3000 |
| Extrait de myrtilles à 30 % de polyphénols totaux en poids | 3500-3600 |
| Extrait polyphénolique à 50 % en poids de polyphénols totaux riche en phloridzine selon le procédé de l'invention | 4000-4500 |
| Extrait polyphénolique à 90 % en poids de polyphénols totaux riche en phloridzine selon le procédé de l'invention | 6000-6500 |
| Extrait polyphénolique à 90 % en poids de polyphénols totaux riche en phlorétine selon le procédé de l'invention | 5800-6300 |

D'après les résultats ci-dessus, les extraits phénoliques extraits de la pomme Malus sylvestris Mill. selon le procédé de l'invention possèdent une activité anti-radicalaire ou anti-oxydante largement supérieure à celle des produits connus pour leur activité anti-oxydante testés en référence.

### Exemple 5 : Effets anti-radicalaires des fractions polyphénoliques en poudre

La génotoxicité d'une substance est définie comme la capacité de celle-ci à générer des lésions sur de l'ADN génomique ou plasmidique. Les agents génotoxiques peuvent être de nature exogène (rayonnements ultraviolets, radiations ionisantes, substances chimiques...) ou de nature endogène (radicaux libres produits par le métabolisme cellulaire...).

Les effets anti-radicalaires des fractions polyphénoliques riches en phloridzine obtenues selon le procédé de l'invention sont mesurés *in vitro* par le test "3D" (**D**amaged **D**NA **D**etection, Analytical Biochemistry, (1995), pp 37-42).

Le principe est basé sur la réparation des lésions de l'ADN, l'ADN lésé étant utilisé comme marqueur d'efficacité protectrice des molécules à tester vis-à-vis des radicaux libres OH° : la diminution des lésions observées sur l'ADN est corrélée au piégeage des radicaux libres. Au cours de l'étape de régénération, un marqueur (nucléotide marqué à la biotine) est incorporé à l'ADN et cette incorporation, reflet quantitatif du nombre de lésions réparées, est ensuite révélée par chimiluminescence.

Ce test effectué sur de l'ADN plasmidique permet la détection d'agents génotoxiques à l'étape précoce du processus lésion - mutation - cancérisation. Les molécules ou mélanges présentant des propriétés anti-oxydantes ou anti-radicalaires, et donc pouvant protéger une cellule humaine des dommages oxydatifs dûs aux espèces oxygénées réactives (EOR) peuvent ainsi être détectés.

Le protocole est le suivant :
- L'ADN cible est adsorbé sur des puits sensibilisés puis incubé avec des agents génotoxiques en présence ou non de l'extrait polyphénolique à tester (solubilisé dans une solution d'éthanol à 2 % ou dans le DMSO) ou en présence de catéchine (contrôle). Dans ce test, le radical OH° produit par scission homolytique de l'eau oxygénée est utilisé comme agent génotoxique lésant l'ADN. L'eau oxygénée est ajoutée dans les puits qui sont ensuite irradiés par des rayons UVB (700 J/m²). De plus, un contrôle positif de réparation consistant en un ADN plasmidique pré-endommagé aux rayonnements ultraviolets C est ajouté.
- Une étape de lavage par de l'eau ultrapure puis par une solution de dioxane à 20 % dans de l'eau ultrapure pendant 5 min. à 30 °C sous agitation douce permet d'éliminer les interactions non spécifiques entre l'échantillon et le test, dues à la présence de macromolécules telles que les protéines pouvant interférer sur le signal de réparation.
- Une étape de réparation est réalisée en présence d'extraits cellulaires purifiés (qui renferment les enzymes actives des différentes voies de réparation des lésions de l'ADN) et de nucléotides modifiés (dUTP) couplés à la biotine. La réparation des lésions implique une phase d'excision des lésions puis une resynthèse du fragment d'ADN ou de bases excisées. Au cours de l'étape de synthèse réparatrice, les nucléotides modifiés sont ainsi incorporés dans l'ADN.
- Une étape de reconnaissance des sites de fixation des nucléotides sur l'ADN par fixation sur la biotine portée par les nucléotides, d'une molécule d'avidine couplée à une molécule de peroxydase.
- Une étape de révélation de la réaction par addition d'un substrat chemiluminescent de la peroxydase. Le signal lumineux observé et lu au luminomètre est proportionnel à la quantité de lésions réparées. Un effet dose est observé dans la limite de 1 à 15 lésions pour 6 kilobases et ce, pour la plupart des lésions.

Le pourcentage de protection observé en présence de EOR est calculé comme la baisse relative de l'effet lésionnel dû aux espèces OH°, soit :
RLU oxydant - RLU (oxydant + échantillon) / RLU oxydant
   avec RLU = relative light units (unités arbitraires de quantité de lumière).

Le pourcentage de protection spécifique est corrigé du pourcentage d'inhibition non spécifique mesuré sur les échantillons pré-lésés aux UVC. Il est le reflet de l'activité anti-radicalaire due aux seuls extraits ajoutés.

IC50 est la concentration en mg/mL de produit testé qui donne 50 % d'activité protectrice de l'agent génotoxique OH°. Plus elle est faible, meilleur est le produit testé vis-à-vis de l'activité anti-oxydante.

| Produit testé | Concentration (en mg / mL) | Protection spécifique (en %) | IC50 (en mg/mL) |
|---|---|---|---|
| Catéchine (molécule de référence) | 1.00 | 65 | 0.24 |
| | 0.25 | 52 | |
| | 0.06 | 28 | |
| | 0.016 | 16 | |
| Epicatéchine (molécule de référence) | 1 | 83 | 0.06 |
| | 0.25 | 71 | |
| | 0.10 | 59 | |
| | 0.010 | 37 | |
| Extrait phénolique à 50 % de polyphénols totaux riche en phloridzine selon le procédé de l'invention | 0.10 | 75 | 0.05 |
| | 0.01 | 41 | |
| | 0.001 | 17 | |
| | 0.0001 | 5 | |
| Extrait phénolique à 90 % de polyphénols totaux riche en phloridzine selon le procédé de l'invention | 0.10 | 95 | 0.005 |
| | 0.01 | 64 | |
| | 0.001 | 27 | |
| | 0.0001 | 7 | |
| Extrait phénolique à 90 % de polyphénols totaux riche en phlorétine selon le procédé de l'invention | 0.10 | 60 | 0.075 |
| | 0.01 | 23 | |
| | 0.001 | 10 | |
| | 0.0001 | 1 | |

Les trois échantillons présentent un effet protecteur vis-à-vis de la formation de lésions sur l'ADN par les radicaux OH°. Ils présentent ainsi une excellente activité anti-radicalaire, supérieure à celle de l'épicatéchine et très supérieure à celle de la catéchine, les deux molécules de référence elles-mêmes reconnues pour leur bonne capacité anti-radicalaire. Un effet dose-réponse est visible.

Les concentrations donnant 50 % d'effets protecteurs sont basses : il faut environ 5 fois moins d'extrait polyphénolique à 50 % que de catéchine pour le même niveau de protection, et environ 50 fois moins d'extrait à 90 % de polyphénols riches en phloridzine. De faibles concentrations en extraits polyphénoliques tels que décrits dans l'invention suffisent à entraîner une diminution nette de l'effet lésionnel des radicaux libres. Les fractions polyphénoliques selon l'invention sont donc des agents anti-radicalaires puissants qui protègent *in vitro* l'ADN des effets génotoxiques du radical OH°.

### Exemple 6 : Effets anti-oxydants des fractions polyphénoliques en poudre

Les effets anti-oxydants des fractions polyphénoliques riches en phloridzine obtenues selon le procédé de l'invention sont mesurés sur de l'ADN plasmidique selon le principe de test "3D" décrit précédemment. La méthodologie est la même à la seule différence que, dans ce test, l'agent génotoxique qui lèse l'ADN est l'oxygène singulet ¹O₂ produit par éclairement du bleu de méthylène.

Le principe est basé sur la réparation des lésions de l'ADN, l'ADN lésé étant utilisé comme marqueur d'efficacité protectrice de molécules diverses vis-à-vis de l'oxygène singulet ¹O₂

Le bleu de méthylène ainsi éclairé et dilué à 4 ng/mL dans l'eau ultra-pure est introduit dans les puits qui sont ensuite irradiés par de la lumière blanche (2 x 75 W) pendant 20 minutes. La molécule de référence utilisée est la silymarine ou silibinine (SIGMA), mélange de flavono-lignanes anti-hépatotoxique extrait et purifié du *Silybum marianum* bien connu pour son activité détoxifiante vis-à-vis de l'oxygène.

Les résultats sont les suivants :

| Produit testé | Concentration (en mg/mL) | Protection spécifique (en %) |
|---|---|---|
| Silymarine | 1 0.1 | 74.6 0 |
| Extrait phénolique à 50 % de polyphénols totaux riche en phloridzine selon le procédé de l'invention | 1. 0.1 | 85.8 0 |
| Extrait phénolique à 90 % de polyphénols totaux riche en phloridzine selon le procédé de l'invention | 1 0.1 | 90 81 |
| Jus concentré de "pommes industries" titrant 1.59 mg PPT/g (polyphénols exprimés en équivalent phloridzine) | 100 10 1 | 80.3 77.1 0 |
| PPT : polyphénols totaux. | | |

Les extraits obtenus selon l'invention présentent une activité anti-oxydante environ 100 fois supérieure à celle du concentré de jus de pomme (qui a lui-même une activité non négligeable).

Leur activité est comparable à celle de la silymarine pour l'extrait phénolique à 50 % en poids de polyphénols totaux riche en phloridzine et dix fois supérieure à celle de la silymarine pour l'extrait phénolique à 90 % en poids de polyphénols totaux riche en phloridzine selon le procédé de l'invention

Les fractions polyphénoliques selon l'invention sont donc des anti-radicalaires puissants qui peuvent jouer un rôle important dans la protection de l'organisme soumis à l'action d'espèces oxygénées réactives formées par le métabolisme cellulaire.

### Exemple 8 : Effet photo-génoprotecteur des fractions polyphénoliques en poudre

Le rôle protecteur anti-ultraviolets des fractions polyphénoliques riches en phloridzine selon l'invention estmesuré sur de l'ADN plasmidique selon le principe de test " 3D" *in vitro* décrit précédemment.

Le principe est basé sur la mesure de la quantité de lésions que subit l'ADN, l'ADN lésé étant utilisé comme marqueur d'efficacité protectrice de molécules à tester vis-à-vis des ultraviolets.

Les produits potentiellement photo-protecteurs à tester sont dilués dans de l'éthanol pur ou de l'eau ultrapure puis déposés dans une plaque transparente aux ultraviolets. Cette plaque est ensuite superposée à celle contenant l'ADN plasmidique adsorbé. L'ensemble est irradié par des rayons ultraviolets B à une puissance de 50 KJ/m². Le produit de référence utilisé est un produit du commerce reconnu pour son action protectrice vis-à-vis des ultraviolets, le Parsol MCX de la société Roche.

Une inhibition non spécifique (en absence d'ultraviolets) du signal de réparation n'est pas possible dans ce test, car à aucun moment le produit à l'essai n'est en contact direct avec l'ADN adsorbé.

IC50 est la concentration en mg/mL de produit testé qui donne 50 % d'activité protectrice des ultraviolets B. Plus elle est faible, meilleur est le produit testé vis-à-vis de l'activité anti-oxydante.

Les résultats sont les suivants :

| | Concentration (en mg / mL) | Protection spécifique (en %) | IC 50 (en mg/mL) |
|---|---|---|---|
| Parsol MCX (Roche) | 1 | 64.9 | |
| | 0.1 | 69.1 | 0.03 |
| | 0.01 | 39.2 | |
| Extrait phénolique à 50 % en poids de polyphénols totaux riche en phloridzine selon le procédé de l'invention | 1 | 49.7 | |
| | 0.1 | 23.0 | 1.00 |
| | 0.01 | 0 | |
| Extrait phénolique à 90 % en poids de polyphénols totaux riche en phloridzine selon le procédé de l'invention | 1 | 70.1 | |
| | 0.1 | 38.4 | 0.40 |
| | 0.01 | 21.6 | |
| | 0.001 | 13.6 | |
| Jus concentré de 'pommes industries' titrant 1.59 mg PPT/g (polyphénols exprimés en équivalent phloridzine) | 100 | 59.0 | |
| | 10 | 44.5 | |
| | 1 | 21.0 | 33 |
| | 0.1 | 40.2 | |
| | 0.01 | 37.9 | |

Les extraits polyphénoliques, bien que moins photo-protecteurs que le Parsol, présentent néanmoins une action anti-ultraviolets B importante.

On peut donc conclure que les extraits polyphénoliques tels que décrits dans la présente invention ont un effet photo-génoprotecteur important vis à vis des ultraviolets B, entre 30 et 80 fois supérieur à celui d'un jus concentré de pomme industriel connu.

## Revendications

1. Fraction issue de fruit de la famille des *Rosaceae* **caractérisée par le fait qu'**elle comprend au moins 20 % de polyphénols comprenant au moins 10 % en poids de phloridzine par rapport aux polyphénols totaux.

2. Fraction selon la revendication 1 **caractérisée par le fait qu'**elle contient de 10 à 70 % en poids de phloridzine par rapport aux polyphénols totaux.

3. Fraction selon l'une quelconque des revendications 1 ou 2 **caractérisée par le fait qu'**elle contient de l'acide chlorogénique.

4. Fraction selon l'une quelconque des revendications 1 à 3 dont le rapport en poids de phloridzine par rapport au poids d'acide chlorogénique est supérieur ou égal à 1.

5. Fraction selon l'une quelconque des revendications 1 à 4 **caractérisée par le fait qu'**elle contient de l'épicatéchine.

6. Fraction selon l'une quelconque des revendications 1 à 5 **caractérisée par le fait qu'**elle contient de la procyanidine B2.

7. Fraction selon l'une quelconque des revendications 1 à 6 dont le rapport en poids de phloridzine par rapport au poids d'épicatéchine est supérieur à 9.

8. Fraction selon l'une quelconque des revendications 1 à 7 **caractérisée par le fait qu'**elle contient de la quercitrine.

9. Fraction selon l'une quelconque des revendications 1 à 8 **caractérisée par le fait qu'**elle contient de l'acide p-coumarique.

10. Fraction selon l'une quelconque des revendications 1 à 9 **caractérisée par le fait qu'**elle contient de l'acide cafféique dans des quantités telles que le rapport en poids de phloridzine par rapport au poids d'acide cafféique est supérieure à 4.

11. Fraction selon la revendication 10 dont la proportion en poids d'acide cafféique représente moins de 1 % des polyphénols totaux.

12. Fraction selon l'une quelconque des revendications 3 à 11 **caractérisée par le fait qu'**elle contient de la phlorétine.

13. Fraction selon l'une quelconque des revendications 3 à 11 **caractérisée par le fait que** les dihydrochalcones (phloridzine et phlorétine) sont présentes dans des proportions supérieures ou égales à 40 % en poids par rapport aux acides hydroxycinnamiques (acide cafféique, acide chlorogénique, acide p-coumarique).

14. Composition selon l'un quelconque des revendications 1 à 13 **caractérisée par le fait qu'**elle contient au moins 20 % en poids de polyphénols totaux dont 10 % au moins de phloridzine, de l'acide chorogénique, de l'épicatéchine, de la procyanidine B2, de la quercitrine, de l'acide p-coumarique et de l'acide cafféique.

15. Fraction selon l'une quelconque des revendications 1 à 14 **caractérisée par le fait qu'**elle contient plus de 50 % en poids de polyphénols totaux.

16. Fraction selon l'une quelconque des revendications 1 à 15 **caractérisée par le fait qu'**elle se présente sous forme de poudre.

17. Fraction selon la revendication 16 **caractérisée par le fait qu'**elle contient au moins 10 % de phloridzine par gramme de poudre.

18. Fraction selon la revendication 16 **caractérisée par le fait qu'**elle contient de 10 à 70 % de phloridzine par gramme de poudre.

19. Procédé de préparation d'une fraction selon l'une quelconque des revendications 1 à 18 **caractérisé par le fait que** l'on procède, à partir de pommes broyées, à une ou plusieurs extractions solide/liquide, l'extrait solide est ensuite séché, puis on procède à de nouvelles extractions par des solvants organiques pour obtenir la fraction polyphénolique.

20. Procédé de préparation d'une fraction selon l'une quelconque des revendications 1 à 18 **caractérisé par le fait que** l'on procède, à partir de pommes broyées, à une ou plusieurs extractions solide/liquide, l'extrait solide est ensuite liquéfié enzymatiquement pour obtenir un extrait liquide qui est traité par chromatographie pour obtenir la fraction polyphénolique.

21. Procédé de préparation d'une fraction selon l'une des revendications 19 ou 20 **caractérisé par le fait que** l'on procède sur l'extrait solide à une étape de dé-cirage.

22. Procédé de préparation d'une fraction selon l'une quelconque des revendications 19 à 21 **caractérisé par le fait que** l'on procède à un séchage final.

23. Procédé selon l'une quelconque des revendications 19 à 22 **caractérisé par le fait que** l'on procède à une extraction de pommes matures.

24. Procédé selon l'une quelconque des revendications 19 à 23 **caractérisé par le fait que** l'on procède à une extraction de la pomme Malus sylvestris Mill.

25. Fraction selon l'une quelconque des revendications 1 à 18 **caractérisée par le fait qu'**elle est susceptible d'être obtenue par le procédé tel que défini dans l'une quelconque des revendications 19 à 24.

26. Composition alimentaire ou nutraceutique comprenant une composition telle que définie dans l'une quelconque des revendications 1 à 18 et 25.

27. Utilisation de la fraction telle que définie dans l'une quelconque des revendications 1 à 18 et 25 comme agent de protection vis-à-vis des rayonnements ultraviolets.

28. Utilisation de la fraction telle que définie dans l'une quelconque des revendications 1 à 18 et 25 comme agent anti-oxydant.

29. Composition cosmétique **caractérisée par le fait qu'**elle contient une fraction telle que définie dans l'une quelconque des revendications 1 à 18 et 25.

## Patentansprüche

1. Fraktion aus der Frucht der Familie der *Rosaceae*, **dadurch** charakterisiert, dass sie mindestens 20% von Polyphenolen umfasst, umfassend mindestens 10 Gewichtsprozent von Phlorizin im Verhältnis zu den Gesamtpolyphenolen.

2. Fraktion nach Anspruch 1, **dadurch** charakterisiert, dass sie 10 bis 70 Gewichtsprozent von Phlorizin im Verhältnis zu den Gesamtpolyphenolen enthält.

3. Fraktion nach einem der Ansprüche 1 oder 2, **dadurch** charakterisiert, dass sie Chlorogensäure enthält.

4. Fraktion nach einem der Ansprüche 1 bis 3, wobei das Gewichtsverhältnis von Phlorizin im Verhältnis zum Gewicht der Chlorogensäure größer oder gleich 1 ist.

5. Fraktion nach einem der Ansprüche 1 bis 4, **dadurch** charakterisiert, dass sie Epicatechin enthält.

6. Fraktion nach einem der Ansprüche 1 bis 5, **dadurch** charakterisiert, dass sie Procyanidin B2 enthält.

7. Fraktion nach einem der Ansprüche 1 bis 6, wobei das Gewichtsverhältnis von Phlorizin im Verhältnis zum Gewicht von Epicatechin größer als 9 ist.

8. Fraktion nach einem der Ansprüche 1 bis 7, **dadurch** charakterisiert, dass sie Quercitrin enthält.

9. Fraktion nach einem der Ansprüche 1 bis 8, **dadurch** charakterisiert, dass sie p-Coumarinsäure enthält.

10. Fraktion nach einem der Ansprüche 1 bis 9, **dadurch** charakterisiert, dass sie Koffeinsäure in solchen Mengen enthält, dass das Gewichtsverhältnis an Phlorizin im Verhältnis zum Gewicht der Koffeinsäure größer als 4 ist.

11. Fraktion nach Anspruch 10, wobei das Gewichtsverhältnis von Koffeinsäure weniger als 1 % der gesamten Polyphenole darstellt.

12. Fraktion nach einem der Ansprüche 3 bis 11, **dadurch** charakterisiert, dass sie Phloretin enthält.

13. Fraktion nach einem der Ansprüche 3 bis 11, **dadurch** charakterisiert, dass die Dihydrochalcone (Phlorizin und Phloretin) in Proportionen vorhanden sind, welche größer oder gleich 40 Gewichtsprozent sind, verglichen mit den Hydroxy-Zimtsäuren (Koffeinsäure, Chlorogensäure, p-Coumarinsäure).

14. Zusammensetzung nach einem der Ansprüche 1 bis 13, **dadurch** charakterisiert, dass sie mindestens 20 Gewichtsprozent an Gesamtpolyphenolen enthält, davon zumindest 10% an Phlorizin, Chlorogensäure, Epicatechin, Procyanidin B2, Quercitrin, p-Coumarinsäure und Koffeinsäure.

15. Fraktion nach einem der Ansprüche 1 bis 14, **dadurch** charakterisiert, dass sie mehr als 50 Gewichtsprozent an Gesamtpolyphenolen enthält.

16. Fraktion nach einem der Ansprüche 1 bis 15, **dadurch** charakterisiert, dass sie als Puderform vorliegt.

17. Fraktion nach Anspruch 16, **dadurch** charakterisiert, dass sie mindestens 10% Phlorizin pro Gramm Puder enthält.

18. Fraktion nach Anspruch 16, **dadurch** charakterisiert, dass sie 10 bis 70% Phlorizin pro Gramm Puder enthält.

19. Verfahren zur Herstellung einer Fraktion nach einem der Ansprüche 1 bis 18, **dadurch** charakterisiert, dass man, ausgehend von zerkleinerten Äpfeln, eine oder mehrere Fest/Flüssig-Extraktionen vornimmt, das solide Extrakt anschließend trocknet und dann weitere Extraktionen mit Hilfe von organischen Lösungsmitteln vornimmt, um die Polyphenolfraktion zu erhalten.

20. Verfahren zur Herstellung einer Fraktion nach einem der Ansprüche 1 bis 18, **dadurch** charakterisiert, dass man, ausgehend von zerkleinerten Äpfeln, eine oder mehrere Fest/Flüssig-Extraktionen vornimmt, das feste Extrakt anschließend enzymatisch verflüssigt, um einen flüssigen Extrakt zu erhalten, welcher der Chromatographie unterzogen wird, um die Polyphenolfraktion zu erhalten.

21. Verfahren zur Herstellung einer Fraktion nach einem der Ansprüche 19 oder 20, **dadurch** charakterisiert, dass man nach der festen Extraktion einen Entwachsungsschritt vornimmt.

22. Verfahren zur Herstellung einer Fraktion nach einem der Ansprüche 19 bis 21, **dadurch** charakterisiert, dass man eine letzte Trocknung vornimmt.

23. Verfahren nach einem der Ansprüche 19 bis 22, **dadurch** charakterisiert, dass man eine Extraktion von reifen Äpfeln vornimmt.

24. Verfahren nach einem der Ansprüche 19 bis 23, **dadurch** charakterisiert, dass man eine Extraktion des Apfels *Malus sylvestris Mill.* vornimmt.

25. Fraktion nach einem der Ansprüche 1 bis 18, **dadurch** charakterisiert, dass sie mit Hilfe des Verfahrens, wie definiert in einem der Ansprüche 19 bis 24, erhalten werden kann.

26. Zusammensetzung aus Nahrungsmitteln oder gesundheitsfördernden Nahrungsmitteln umfassend eine Zusammensetzung wie definiert in einem der Ansprüche 1 bis 18 und 25.

27. Verwendung der Fraktion wie definiert in einem der Ansprüche 1 bis 18 und 25, als Schutzagens gegen ultraviolette Strahlen.

28. Verwendung der Fraktion wie definiert in einem der Ansprüche 1 bis 18 und 25 als Antioxidans-Mittel.

29. Kosmetische Zusammensetzung **dadurch** charakterisiert, dass sie eine Fraktion wie definiert in einem der Ansprüche 1 bis 18 und 25 enthält.

## Claims

1. Fraction obtained from fruit of the *Rosaceae* family, **characterized in that** it comprises at least 20% of polyphenols comprising at least 10% by weight of phloridzin relative to the total polyphenols.

2. Fraction according to Claim 1, **characterized in that** it contains from 10% to 70% by weight of phloridzin relative to the total polyphenols.

3. Fraction according to either of Claims 1 and 2, **characterized in that** it contains chlorogenic acid.

4. Fraction according to any one of Claims 1 to 3, in which the weight ratio of phloridzin relative to the weight of chlorogenic acid is greater than or equal to 1.

5. Fraction according to any one of Claims 1 to 4, **characterized in that** it contains epicatechin.

6. Fraction according to any one of Claims 1 to 5, **characterized in that** it contains procyanidin B2.

7. Fraction according to any one of Claims 1 to 6, in which the weight ratio of phloridzin relative to the weight of epicatechin is greater than 9.

8. Fraction according to any one of Claims 1 to 7, **characterized in that** it contains quercitrin.

9. Fraction according to any one of Claims 1 to 8, **characterized in that** it contains p-coumaric acid.

10. Fraction according to any one of Claims 1 to 9, **characterized in that** it contains caffeic acid in amounts such that the weight ratio of phloridzin relative to the weight of caffeic acid is greater than 4.

11. Fraction according to Claim 10, in which the weight proportion of caffeic acid represents less than 1% of the total polyphenols.

12. Fraction according to any one of Claims 3 to 11, **characterized in that** it contains phloretin.

13. Fraction according to any one of Claims 3 to 11, **characterized in that** the dihydrochalcones (phloridzin and phloretin) are present in proportions of greater than or equal to 40% by weight relative to the hydroxycinnamic acids (caffeic acid, chlorogenic acid and p-coumaric acid).

14. Composition according to any one of Claims 1 to 13, **characterized in that** it contains at least 20% by weight of total polyphenols, including at least 10% of phloridzin, chlorogenic acid, epicatechin, procyanidin B2, quercitrin, p-coumaric acid and caffeic acid.

15. Fraction according to any one of Claims 1 to 14, **characterized in that** it contains more than 50% by weight of total polyphenols.

16. Fraction according to any one of Claims 1 to 15, **characterized in that** it is in powder form.

17. Fraction according to Claim 16, **characterized in that** it contains at least 10% phloridzin per gram of powder.

18. Fraction according to Claim 16, **characterized in that** it contains from 10% to 70% of phloridzin per gram of powder.

19. Process for preparing a fraction according to any one of Claims 1 to 18, **characterized in that**, starting with crushed apples, one or more solid/liquid extractions is carried out, the solid extract is then dried, after which further extractions with organic solvents are carried out to obtain the polyphenolic fraction.

20. Process for preparing a fraction according to any one of Claims 1 to 18, **characterized in that**, starting with crushed apples, one or more solid/liquid extractions is carried out, the solid extract is then enzymatically liquefied to obtain a liquid extract, which is then treated by chromatography to obtain the polyphenolic fraction.

21. Process for preparing a fraction according to either of Claims 19 and 20, **characterized in that** a dewaxing step is performed on the solid extract.

22. Process for preparing a fraction according to any one of Claims 19 to 21, **characterized in that** a final drying is performed.

23. Process according to any one of Claims 19 to 22, **characterized in that** an extraction of ripe apples is performed.

24. Process according to any one of Claims 19 to 23, **characterized in that** an extraction of Malus sylvestris Mill. apple is performed.

25. Fraction according to any one of Claims 1 to 18, **characterized in that** it may be obtained by the process as defined in any one of Claims 19 to 24.

26. Dietary or nutraceutical composition comprising a composition as defined in any one of Claims 1 to 18 and 25.

27. Use of the fraction as defined in any one of Claims 1 to 18 and 25, as an agent for protecting against ultraviolet radiation.

28. Use of the fraction as defined in any one of Claims 1 to 18 and 25, as an antioxidant.

29. Cosmetic composition, **characterized in that** it contains a fraction as defined in any one of Claims 1 to 18 and 25.
